# EUROPEAN PATENT APPLICATION

(11) **EP 1 759 596 A1**
(43) Date of publication of application: **07.03.2007**
(21) Application number: 04807577.4
(22) Date of filing: 22.12.2004
(51) Int. Cl.: A23L 1/30

(54) **PROCESS FOR PRODUCING WHITISH BANABA EXTRACT, WHITISH BANABA EXTRACT AND FOOD OR DRINK CONTAINING WHITISH BANABA EXTRACT**

(30) Priority: 21.04.2004 JP 2004126055; 21.04.2004 JP 2004126056; 21.04.2004 JP 2004126057; 21.04.2004 JP 2004126058; 21.04.2004 JP 2004126060
(71) Applicant: Use-Techno Corporation, Fukuchiyama-shi, Kyoto 6200055 (JP)
(72) Inventor: MATSUYAMA, Futoshi, 6208502 (JP)
(74) Representative: Ouzman, Beverley Nicola Claire
(86) International application number: PCT/JP2004/019220
(87) International publication number: WO 2005/102072

(57) **Abstract**

The present invention relates to a method of producing a whitish banaba extract having a high corosolic acid content and a whitish appearance, and to a food or drink product containing the whitish banaba extract. According to the present invention, there is provided a method of producing a banaba extract having a high corosolic acid content and a whitish appearance. In addition, using the method, there can be provided a whitish banaba extract having a high corosolic acid content and a whitish appearance. Moreover, using the whitish banaba extract, there can be provided a food or drink product having a high corosolic acid content and a whitish appearance.

## Description

### Technical Field

The present invention relates to a method of producing a whitish banaba extract, a whitish banaba extract obtainable by the method, and a food or drink product containing the whitish banaba extract.

### Background Art

It is known that a banaba extract containing a given amount of corosolic acid, which has the effect of suppressing blood glucose increase or lowering blood glucose levels, can be obtained by extracting a dried banaba leaf with hot water or alcohol (Patent document 1).
Patent document 1: Japanese Patent Application Laid-Open No. 2000-169384

### Disclosure of the Invention

### Problem to be Solved by the Invention

It is thought that the pharmacological effect of suppressing blood glucose increase or lowering blood glucose levels is due to the corosolic acid in banaba extract. However, since corosolic acid is lipophilic and it is contained in a banaba leaf to the extent of only about 0.01% to 0.5%, it is difficult to increase the corosolic acid content even by hot water extraction. Also, the color of banaba extract obtained by ethanol extraction is brown, and it has a distinct odor.

Thus, although it is desirable to add banaba extract to food or drink not only to suppress blood glucose increase or to lower blood glucose levels, but also to decrease triglyceride and cholesterol levels and thereby to prevent arteriosclerosis, a product suitable for actual consumption has not yet been obtained, because the corosolic acid content of banaba extract is low and the extract is colored.

It is therefore an object of the present invention to provide a method of producing a banaba extract having a high corosolic acid content and a whitish appearance. In addition, it is also an object of the present invention to provide a whitish banaba extract obtainable by the method. Moreover, it is also an object of the present invention to provide a food or drink product containing the whitish banaba extract, and having a high corosolic acid content and a whitish appearance.

### Means for Solving the Problem

In order to achieve the object, the present invention provides a method of producing a whitish banaba extract comprising a pressurizing step of pressurizing a soaked material obtained by soaking banaba tissue in water, and an extraction step of extracting banaba tissue subjected to the pressurizing step with a mixture of organic solvent and water, or with organic solvent.

In the extraction method of obtaining banaba extract according to the prior art (hot water extraction method), a hot water extract was used as a corosolic acid-containing banaba extract, whereas in the production method of the present invention, a water extract obtained by pressurizing is not used, and a banaba extract having a high corosolic acid content is prepared using the extraction residue. In other words, a corosolic acid-containing banaba extract is derived from banaba tissue.

Since a large amount of colored tannin dissolves out in a water extract obtained in a pressurizing step, banaba tissue which has undergone a pressurizing step has a lighter color than the hot water extract of the prior art. Therefore, using this banaba tissue, it is possible to obtain a banaba extract which has become whitish. Also, since a sufficient amount of corosolic acid is left in banaba tissue which has undergone a suitable pressurizing step, the obtained whitish banaba extract has a high corosolic acid content.

In the production method of the present invention, banaba tissue which has undergone a pressurizing step is extracted with a predetermined extraction solvent (mixture of organic solvent and water, or organic solvent), and by using this extraction method, the amount of corosolic acid contained in a banaba extract can be increased.

In the production method of the present invention, a soaked material obtained by soaking banaba tissue in water for at least 5 hours is preferably used. In addition, the pressurizing step is preferably performed at 0°C to 120°C, pressurizing being more preferably performed while heating. Pressurizing is preferably performed at 1 to 3 atm. In this way, large amounts of colored components (tannins etc.) can be removed in the pressurizing step.

The aforementioned effects become marked by performing pressurizing more than once in the pressurizing step. Tannin simultaneously obtained in the extraction may be used in drinks such as banaba tea, as well as in cosmetics and health foods.

The banaba tissue subjected to the pressurizing step is most preferably dried banaba tissue obtained by removing the water used in the pressurizing step. The organic solvent used for the extraction is preferably alcohol and/or hydrocarbon organic solvent. This makes it possible to easily obtain a whitish banaba extract containing a larger amount of corosolic acid.

In the production method of the present invention, extraction may also be performed more than once in the extraction step. This makes it possible to achieve the aforementioned effects with more certainty. In this case, an extraction with a mixture of alcohol and water, and an extraction with a mixture of alcohol and hydrocarbon organic solvent may be performed separately to obtain different extracts depending on the extraction solvent. For example, by using a mixture of alcohol and hydrocarbon organic solvent (e.g. hexane), chlorophyll can be extracted efficiently. The banaba tissue used is preferably a banaba leaf, because it contains a large amount of corosolic acid.

The present invention also provides a food product containing the aforesaid whitish banaba extract, and containing an edible material which contains carbohydrate or plant fiber. The present invention also provides a food product containing the aforesaid whitish banaba extract and containing an oil/fat. The present invention further provides a banaba extract-containing drink product containing the aforesaid whitish banaba extract and containing a drink.

The banaba extract added in the present invention is the whitish banaba extract obtained by the production method comprising the aforesaid steps. In the food product of the present invention containing the whitish banaba extract, and containing an edible material which contains carbohydrate or plant fiber, or in the food product of the present invention containing the whitish banaba extract and containing an oil/fat, the original color of the edible material or the oil/fat is not impaired by the banaba extract, while the corosolic acid content can be increased. In the banaba extract-containing drink product of the present invention containing the whitish banaba extract and containing a drink, the original color of the drink is not impaired by the banaba extract, while the corosolic acid content can be increased. Therefore, the food product or drink product of the present invention exerts the effect of suppressing blood glucose increase or lowering blood glucose levels when consumed as a food or drink.

Since the banaba extract is derived from a natural product, it is excellent also in safety. In particular, in the case where the food product of the present invention contains an oil/fat, it has anti-obesity and antioxidant properties, and is healthier and safer than oil/fat-containing food products which do not contain the whitish banaba extract.

Plant fibers generally suppress absorption of glucose, and are thought to suppress blood glucose increase even when consumed alone, but this suppression effect only endures for a short time after eating. However, the food product of the present invention containing a plant fiber-containing edible material makes it possible for the effect of suppressing blood glucose increase to endure not only for 30 minutes after eating, but also for 2 to 3 hours. In addition, it also exhibits the synergistic effects of, for example, decreasing triglyceride levels, preventing side-effects such as constipation and diarrhea, and reducing cholesterol levels.

An edible material containing a processed plant fiber such as indigestible dextrin takes time to digest, and is absorbed gradually, and therefore it activates gut motility. Therefore, when the food product of the present invention containing such a plant fiber-containing edible material and containing the whitish banaba extract is consumed, the activated gut effectively absorbs active ingredients of the whitish banaba extract such as corosolic acid, and the effect of suppressing blood glucose increase is enhanced.

The banaba extract-containing drink product of the present invention prevents sharp rises in blood glucose levels caused by the high absorbability of nutrients in a drink to which the whitish banaba extract is added, and prevents rises in blood glucose levels caused by the delay of insulin secretion following an increase in blood glucose. It also suppresses triglyceride synthesis, and prevents obesity and arteriosclerosis.

The whitish banaba extract is preferably contained to the extent of 0.0001 to 10 parts by weight per 100 parts by weight of the edible material. In this case, the effect of suppressing blood glucose increase or lowering blood glucose levels is enhanced when the food product is consumed as a food.

### Effects of the Invention

According to the present invention, there is provided a method of producing a banaba extract having a high corosolic acid content and a whitish appearance. In addition, using the method, there can be provided a whitish banaba extract having a high corosolic acid content and a whitish appearance. Moreover, using the whitish banaba extract, there can be provided a food or drink product having a high corosolic acid content and a whitish appearance.

### Best Modes for Carrying Out the Invention

Preferred embodiments of the production method of a whitish banaba extract of the present invention will now be described on a step-by-step basis.

### (Pressurizing step)

The banaba tissue used in the pressurizing step of the method of producing a whitish banaba extract is preferably a banaba leaf. The banaba leaf is preferably a mature leaf which has grown for 6 months after sprouting, and is more preferably a whole leaf which has naturally turned red or yellow, or a leaf which has been stimulated by an insect such as an ant.

As the leaf, a whole leaf or a broken leaf may be used. However, considering extraction time and subsequent producing steps, a broken leaf is preferred. From the viewpoint of extraction efficiency, there may be preferred a broken leaf obtained by drying a whole leaf and then cutting it into strips or squares of 1 to 20 mm in width with a cutter. A leaf in such a shape is superior also from the viewpoint of penetration of the extraction solvent and clogging of the extraction film.

In the pressurizing step, banaba tissue such as a dried banaba leaf is soaked in water before pressurizing. The soaking in water may be performed for 5 to 24 hours, and the soaking may be repeated 2 to 10 times. The temperature is preferably 0°C to 50°C. The amount of water relative to dried banaba leaves is preferably 2 to 20 L per 1 kg of leaves. The purpose of the soaking in water is to extract tannins, but since the extracted tannins vary depending on the temperature, the soaking is preferably performed in stages at 0°C to 10°C, 11°C to 30°C, and 31°C to 50°C.

The purpose of the pressurizing step is to remove tannins and chlorophylls from the banaba tissue while leaving the active components intact. From the viewpoint of efficient removal of tannins and chlorophylls, the pressurizing step is preferably performed at 0°C to 120°C (preferably 10°C to 120°C, more preferably 30°C to 120°C, still more preferably 50°C to 120°C), and at 1 to 3 atm (preferably 1 to 2.5 atm, more preferably 1 to 2 atm). Pressurizing under these conditions is preferably performed once or 2 to 5 times. If the pressure is increased in stages when pressurizing is performed, tannins and chlorophylls are removed more efficiently.

The pressurizing step may be performed using a pressure vessel, and if the pressure exceeds 1.5 atm, the pressurizing time is preferably less than 10 minutes. In this case, most preferably, pressurizing is first performed at 1.1 to 1.3 atm, and then performed twice at 1.3 to 1.6 atm. Prolonged extraction leads to extraction of corosolic acid, and therefore it should be avoided.

### (Extraction step)

In the extraction step, the banaba tissue obtained in the pressurizing step is subjected to extraction with a mixture of organic solvent and water, or with organic solvent. The banaba tissue is preferably obtained by removing water by filtration from the mixture of water and banaba tissue obtained in the pressurizing step. The filtered banaba tissue is more preferably dried in the sun or with a drier. After the filtering, moisture is preferably removed by centrifugation, and drying is preferably performed using a warm air current of 30°C to 60°C or a cold air current. For efficient drying, it is preferable to employ an air current with as low a humidity as possible. If drying is performed with a hot air drier exceeding 100°C, the drying time is preferably less than 10 minutes.

The dried banaba tissue is preferably extracted with hexane or with a mixture of hexane and water. Prior to the extraction, tannins and chlorophylls may first be removed with resin or activated carbon. If extraction is performed more than once using the same or different extraction solvents, the extraction step becomes more effective. In this case, the last extraction is preferably performed with alcohol (e.g., 90% to 100% ethanol).

### (Whitish banaba extract)

The whitish banaba extract can be obtained by the extraction. The whitish banaba extract may contain some extraction solvent, but the extraction solvent is preferably removed. As examples of the components of the whitish banaba extract from which the extraction solvent has been removed, there may be mentioned corosolic acid, maslinic acid, tormentic acid, ursolic acid, oleanolic acid, a-amyrin, β-amyrin, asiatic acid, 18β-glycyrrhetinic acid, tannins and hemicellulose. The whitish banaba extract normally contains corosolic acid, which is the main active ingredient, in a proportion of 3% to 50% or more. The whitish banaba extract may be in the form of a liquid, paste or powder. The whitish banaba extract is preferably stored at room temperature or in a refrigerator away from light in a dried state.

The color of the whitish banaba extract obtainable by the production method of the present invention can be measured with a colorimeter. The L value obtained by colorimetric measurement in the case of powder is normally 50 or more, and typically 60 to 70. When the same measurement is used, the L value of a banaba extract obtained by the prior art hot water extraction method is 30 to 40, showing that the color of the whitish banaba extract is much closer to white than the banaba extract mentioned above. The "a" value obtained by colorimetric measurement when the whitish banaba extract is powdered is typically negative (about 0 to -1), and the "b" value is typically positive (about 20 to 30).

Hence, since the color of the whitish banaba extract obtainable by the production method of the present invention is close to white, when it is used as an additive which has the effect of suppressing blood glucose increase or lowering blood glucose levels, the color of a material to which it is added is not impaired. In addition, the odor and taste of the whitish banaba extract are insignificant, and it is therefore perfect as an additive for an edible material containing carbohydrate or plant fiber, an oil/fat or a drink.

With the prior art banaba extract obtained by hot water extraction or alcohol extraction, elimination of tannins or chlorophylls was difficult and its color was very dark, and therefore its use in food products is limited to use in, for example, health food supplement tablets or capsules. Considering this, the whitish banaba extract of the present invention has very wide application.

In the case of the prior art hot water extract, tannins accounted for 10% to 80% or more, and the corosolic acid content was less than 0.001% to 1%, whereas in the case of the alcohol extract, tannins accounted for 10% to 70%, and the corosolic acid content was less than 0.1% to 6%.

However, according to the method of the present invention, the color, odor and taste problems occurring when using a banaba extract in food products can be resolved, and the amount of corosolic acid, which is the active ingredient, can be increased to 2% to 50% or more. This means that a pronounced effect can be expected even from a small amount of banaba extract, and permits a low price for the final product. Therefore, if the whitish banaba extract is added to an edible material containing carbohydrate or plant fiber, such as bread, noodle, confectionery or rice, which are consumed on a daily basis, or to an oil/fat or a drink, only a small amount of banaba extract is enough, and costs can be kept down.

### (Food product containing the whitish banaba extract)

The food product of the present invention is a product obtained by adding the whitish banaba extract to an edible material containing carbohydrate or plant fiber, or to an oil/fat. As examples of the carbohydrate-containing edible material, there may be mentioned noodles such as udon (wheat noodles), pasta, buckwheat noodles and instant noodles; breads such as ordinary bread, sweet breads and French bread; sweets such as ice cream, biscuits, chips, chocolate and cakes; drinks such as Japanese tea, carbonated drinks, coffee, juice and health drinks; spiced foods such as curry sauce and stew; frozen foods such as croquettes, fried shrimps and squid rings; condiments such as sugar, oils/fats, salt, vinegar, soy sauce, miso (soybean paste), sauce, ketchup, mayonnaise and chili sauce; rice products such as rice balls, sushi and fried rice; and cereal foods such as tohu (bean curd), fried bean curd and natto. As examples of the plant fiber-containing edible material, there may be mentioned synthetic and semisynthetic carbohydrate-containing edible materials (e.g., edible starch materials such as indigestible dextrin and isomerized sugar); natural fiber-containing edible materials such as gum, psyllium and konnyaku (konjac); fiber-containing drinks such as teas, powdered green tea, cocoa, vegetable juice and fruit juice; processed cereal materials such as psyllium, yam, buckwheat noodles, Chinese noodles and udon (wheat noodles); plant fiber-containing edible materials containing lotus root or the like; plant fiber-containing marine-derived edible materials such as hijiki, jelly, agar and seaweed; dried or oil/fat-processed, seed- or fruit-derived edible materials such as banana chips, potato chips and dried mangos; processed gourmet edible materials such as cinnamon powder, chocolate, raisins, prunes and figs; processed, seed-derived edible materials such as soybeans, natto, soymilk, okara (bean curd refuse), goma-dohu (sesame bean curd), black beans, peanuts and almonds; confectionery products such as rice crackers, peanut butter, sugared beans and rice cake; and edible cooking materials such as dried gourd shavings, dried radish, dried potatoes, dried shiitake mushrooms and sliced almonds. As examples of the oil/fat, there may be mentioned oil/fat-containing dairy products such as butter, margarine and cheese; confectionery fats such as shortening and whipped cream; household culinary oils such as palm oil, com oil, soybean oil, cottonseed oil, coconut oil, sesame oil, sunflower oil and mixed vegetable oil; and specialty oils/fats such as lard and powdered oils.

The food product of the present invention preferably contains 0.0001 to 10 parts by weight, more preferably 0.001 to 10 parts by weight, still more preferably 0.01 to 10 parts by weight of the whitish banaba extract relative to 100 parts by weight of the edible material containing carbohydrate or plant fiber, or relative to 100 parts by weight of the oil/fat. Most preferably, the amount of the whitish banaba extract is set so that 1 to 50 mg per day of corosolic acid can be consumed.

In order to add the whitish banaba extract to a carbohydrate-containing edible material, the whitish banaba extract may be added during or after production of the carbohydrate-containing edible material.

Corosolic acid, which is the active ingredient in the whitish banaba extract, is lipophilic, and it is insoluble in water. In order to mix it with a plant fiber-containing edible material, the whitish banaba extract is most preferably dissolved in alcohol, crushed to a size of from 2 µm to 100 nm, spray-dried or freeze-dried as appropriate to form a powder, and then mixed with the plant fiber-containing edible material.

In the case where the whitish banaba extract is added to an oil/fat, the concentration of corosolic acid and the composition of the other components are important regarding mixing with the oil/fat, because corosolic acid contained in the whitish banaba extract is a lipophilic compound. The mixing method should be adapted accordingly. If the corosolic acid content in the whitish banaba extract is 95% to 100%, the particle size is preferably reduced to from 2 µm to 100 nm. In order to mix the whitish banaba extract with an oil/fat, the extract may be dissolved in ethanol, the solution mixed with the oil/fat, and then the alcohol evaporated. On the other hand, if the whitish banaba extract contains other components (e.g., tannins and cellulose), the extract is preferably emulsified before the mixing. In this case, solidification with a starch such as dextrin is also effective.

Besides the whitish banaba extract, and an edible material containing carbohydrate or plant fiber, or an oil/fat, the food product of the present invention may further contain other additives, for example, natural colorants, vitamins, minerals, flavorings, preservatives, indigestible dextrin, other extracts derived from plants such as mulberry, loquat and guava, chromium, CoQ10, artificial sweeteners, amino acids, and tannins such as catechin.

### (Banaba extract-containing drink product)

The banaba extract-containing drink product of the present invention is obtained by adding the whitish banaba extract to a drink. As examples of the drink, there may be mentioned teas such as green tea, oolong tea, health tea and black tea; carbonated drinks such as cola and soda pop; dairy drink products such as milk, yoghurt and lactic acid drinks; coffee beverages such as coffee, milk coffee and black coffee; juice drinks such as fruit juice, green soup, vegetable juice and nectar; functional drinks such as isotonic drinks and nutritional supplement drinks; water such as mineral water and distilled water; low-alcohol drinks such as low-alcohol beer and champagne; non-Japanese alcoholic drinks such as wine, beer, whisky, brandy, tequila, vodka and Shaoxing wine; Japanese alcoholic drinks such as Japanese sake, shochu (distilled spirits) and umeshu (plum wine); alcoholic drinks containing them; and nutritional supplement jelly drinks containing jelly and konnyaku (konjac) as the principal ingredients.

The banaba extract-containing drink product of the present invention preferably contains 0.0001 to 10 parts by weight, more preferably 0.001 to 10 parts by weight, still more preferably 0.01 to 10 parts by weight of the whitish banaba extract relative to 100 parts by weight of the drink. Most preferably, the amount of the whitish banaba extract is set so that 1 to 50 mg per day of corosolic acid can be consumed.

Corosolic acid contained in the whitish banaba extract is a lipophilic compound. Therefore, when the extract is mixed with a drink, the mixing method is preferably varied depending on the characteristics of the drink. In addition, the concentration of corosolic acid and the composition of the other components should be considered.

In other words, the addition method is preferably varied according to a drink to which the extract is to be added. For example, the addition method is optimized depending on the type of drink to which the extract is to be added, e.g., colored drinks such as teas; drinks which should be white or transparent, such as milk or soda pop; drinks whose taste is easily affected by an additive, such as cola and vegetable juice; and alcoholic drinks, in which corosolic acid dissolves easily.

If the corosolic acid content in the whitish banaba extract is 60% to 100%, the particle size is preferably reduced to from 5 µm to 10 nm. In this case, it is too fine to be visible, and therefore it can be added to tea drinks, cola and vegetable juice.

In order to mix the whitish banaba extract with a drink, the extract may be dissolved in ethanol, the solution mixed with the drink, and then the ethanol evaporated. On the other hand, if the corosolic acid content in the whitish banaba extract is about 1% to 59%, the extract is preferably first emulsified. If it is added after emulsification, it does not lead to precipitation. In this case, solidification with a starch such as dextrin is also effective.

With respect to alcoholic drinks, in the case of Japanese sake and shochu (distilled spirits), where transparency is important, and light-flavored drinks whose taste is easily affected by an additive, such as beer and champagne, the whitish banaba extract having a high corosolic acid content is preferably added. On the other hand, in the case of drinks having a relatively low alcohol content, and strongly-flavored non-Japanese alcoholic drinks such as wine, whisky (e.g., bourbon) and brandy, the emulsified whitish banaba extract is preferably added.

Besides the whitish banaba extract and a drink, the banaba extract-containing drink product of the present invention may further contain other additives, for example, natural colorants, vitamins, minerals, flavorings, preservatives, indigestible dextrin, other extracts derived from plants such as mulberry, loquat and guava, chromium, CoQ10, artificial sweeteners, amino acids, and tannins such as catechin.

### Examples

First, colorimetric measurements were performed for a banaba extract obtained by hot water extraction (hereinafter referred to as "Sample 1"), and a whitish banaba extract obtained by the production method of the present invention (hereinafter referred to as "Sample 2"). Both samples were obtained from banaba leaves.

Sample 1 or Sample 2 was introduced into a cell for colorimetric powder measurement, and the L value, "a" value and "b" value were measured using a colorimeter (spectrophotometric colorimeter SE 2000, Nippon Denshoku Industries Co., Ltd.). Three samples were used for each of Sample 1 and Sample 2, and the average value was calculated (the reference value was adjusted using a specified white plate).

As a result, for Sample 1, the L value was 35.21, the "a" value was 0.11 and the "b" value was 14.86, whereas for Sample 2, the L value was 65.57, the "a" value was -0.76 and the "b" value was 23.23. Compared to Sample 1, the L value for Sample 2 was higher by about 30, showing that the degree of whiteness for Sample 2 is much higher than that for Sample 1.

In order to numerically evaluate the visual difference between Sample 1 and Sample 2, the color difference between Sample 1 and Sample 2 measured by taking Sample 1 as a reference was 31.39. Also from this value, it is seen that the color of Sample 2 is completely different from that of Sample 1.

Next, food products and drink products containing the whitish banaba extract were prepared using Sample 2.

### (Whitish banaba extract-containing udon (wheat noodle))

Sample 2 was added during production of udon to obtain an udon containing 0.1 parts by weight of Sample 2 per 100 parts by weight of the material for udon. Visual examination of the obtained udon was performed, and it was seen that there was no apparent difference in color compared to ordinary udon. Also, no significant difference in taste was found when the obtained udon was consumed.

### (Whitish banaba extract-containing instant noodle)

Sample 2 was added to the material for an instant noodle (containing plant fiber) to obtain an instant noodle containing the whitish banaba extract. It was confirmed that since the whitish banaba extract used had a very light taste, odor and color, the effect of suppressing blood glucose increase and the anti-obesity effect were obtained without impairing the original color and flavor of the instant noodle.

### (Whitish banaba extract-containing household frying oil/fat)

Sample 2 was dissolved in alcohol solvent, and this was mixed with an oil/fat. The alcohol solvent was eliminated by heating during the mixing to obtain a household frying oil/fat. Visual examination of the obtained oil/fat was performed, and it was seen that the color of the oil/fat used as the material was not impaired. It was also confirmed that the obtained oil/fat can be used as a daily food product which prevents sharp rises in blood glucose levels caused by the high calories of the oil/fat used as the material, and which is useful for prevention of insulin oversecretion caused by the high nutrition, and obesity resulting therefrom.

### (Banaba extract-containing coffee beverage)

Sample 2 was added simultaneously when blending coffee extract, milk, sugar and flavorings, which are materials for a coffee beverage, to obtain a coffee beverage containing the whitish banaba extract. Its color was not different from that of the original coffee beverage. Also, when the obtained banaba extract-containing coffee beverage was consumed, it was confirmed that the taste and odor of the original coffee beverage were not impaired.

### (Banaba extract-containing whisky)

Whisky containing the whitish banaba extract was obtained by adding Sample 2 to whisky. Visual examination of the obtained banaba extract-containing whisky was performed, and it was seen that the color of the whisky used as the material was not impaired. It was also confirmed that the obtained banaba extract-containing whisky prevents sharp rises in blood glucose levels caused by the high calories of the whisky used as the material, and which is useful in decreasing cholesterol levels, suppressing blood pressure increase, and preventing obesity and arteriosclerosis.

### Industrial Applicability

Using the method of producing a whitish banaba extract of the present invention, there is provided a whitish banaba extract having a high corosolic acid content and a whitish appearance. The food product or drink product of the present invention containing the whitish banaba extract exerts the effect of suppressing blood glucose increase or lowering blood glucose levels when consumed as a food or drink.

## Claims

1. A method of producing a whitish banaba extract, comprising:
a pressurizing step of pressurizing a soaked material obtained by soaking banaba tissue in water; and
an extraction step of extracting banaba tissue subjected to said pressurizing step with a mixture of organic solvent and water, or with organic solvent.

2. The method of producing a whitish banaba extract according to claim 1, wherein said soaked material is obtained by soaking banaba tissue in water for at least 5 hours.

3. The method of producing a whitish banaba extract according to claim 1, wherein the banaba tissue subjected to said pressurizing step is dried banaba tissue obtained by removing the water used in said pressurizing step.

4. The method of producing a whitish banaba extract according to claim 1, wherein said pressurizing step is performed at 0°C to 120°C.

5. The method of producing a whitish banaba extract according to claim 1, wherein pressurizing at 1 to 3 atm is performed in said pressurizing step.

6. The method of producing a whitish banaba extract according to claim 1, wherein pressurizing is performed more than once in said pressurizing step.

7. The method of producing a whitish banaba extract according to claim 1, wherein said organic solvent is alcohol and/or hydrocarbon organic solvent.

8. The method of producing a whitish banaba extract according to claim 1, wherein extraction is performed more than once in said extraction step.

9. The method of producing a whitish banaba extract according to claim 8, wherein an extraction with a mixture of alcohol and water, and an extraction with a mixture of alcohol and hydrocarbon organic solvent are performed in said extraction step.

10. The method of producing a whitish banaba extract according to claim 1, wherein said banaba tissue is a banaba leaf.

11. A whitish banaba extract obtainable by the production method according to any one of claims 1 to 10.

12. A food product containing the whitish banaba extract according to claim 11, and containing an edible material which contains carbohydrate or plant fiber.

13. The food product according to claim 12, containing 0.0001 to 10 parts by weight of said whitish banaba extract relative to 100 parts by weight of said edible material.

14. A food product containing the whitish banaba extract according to claim 11, and containing an oil/fat.

15. The food product according to claim 14, containing 0.0001 to 10 parts by weight of said whitish banaba extract relative to 100 parts by weight of said oil/fat.

16. A banaba extract-containing drink product containing the whitish banaba extract according to claim 11, and containing a drink.

17. The banaba extract-containing drink product according to claim 16, containing 0.0001 to 10 parts by weight of said whitish banaba extract relative to 100 parts by weight of said drink.
